# FASCICULE DE BREVET EUROPEEN

(11) **EP 2 915 507 B1**
(45) Date de publication et mention de la délivrance du brevet: **14.06.2017**
(21) Numéro de dépôt: 15157019.9
(22) Date de dépôt: 27.02.2015
(51) Int. Cl.: A61F 2/46, A61F 2/34, A61F 2/30

(54) **dispositif pour prendre et insérer un élément insert**
VORRICHTUNG ZUM GREIFEN UND EINSETZEN EINES EINSATZELEMENTS
APPARATUS FOR CAPTURING AND INSERTING AN INSERT ELEMENT

(30) Priorité: 07.03.2014 FR 1400564
(43) Date de publication de la demande: 09.09.2015
(73) Titulaire: XNOV IP, 1882 Luxembourg (LU)
(72) Inventeur: Biegun, Jean-François, 2900 Porrentry (CH); Biegun, Frédérique, 2900 Porrentry (CH); Loehle, Pascal, 2900 Porrentruy (CH)
(74) Mandataire: Eidelsberg, Olivier Nathan

(56) Documents cités:
- WO-A1-2008/106598
- WO-A1-2011/161166
- US-A1- 2007 219 562
- US-B1- 6 468 281

## Description

La présente invention se rapporte à un dispositif pour insérer un élément insert, notamment en matériau céramique, à l'intérieur d'une cupule elle-même destinée à être reçue dans la cavité cotyloïdienne d'une hanche d'un patient.

On connaît déjà, par exemple de WO 2011/161166, des dispositifs d'insertion comportant une tige, une tête d'impaction fixée à l'extrémité de la tige et destinée à venir en contact avec l'insert et des moyens de préhension destiné à maintenir temporairement l'insert au dispositif d'insertion lorsque la tête d'impaction est en contact avec l'insert, ce dernier étant ensuite, par l'intermédiaire d'un choc ou impaction appliqué à la tige par le chirurgien, adapté de force à la cupule pour y être solidarisé d'une manière telle qu'il est difficile, voire impossible, de l'en ressortir.

Ces dispositifs de l'art antérieur sont compliqués à utiliser et ne garantissent pas de réaliser une impaction à coup sÛr dans la bonne direction. Il arrive souvent soit que l'insert soit mal orienté par rapport à la cupule avant son impaction, soit que l'axe suivant lequel l'impaction est réalisée ne soit pas parfaitement dans la direction qui convient pour solidariser parfaitement l'insert et la cupule. La conséquence peut en être que l'insert soit mal coincé dans la cupule, entraînant sur le long terme des risques de rupture, et les complications associés pour le patient (nouvelle opération pour retirer la cupule défectueuse, qui souvent s'est brisée, et repose d'une cupule).

La présente invention vise à surmonter les inconvénients de l'art antérieur en proposant un dispositif d'insertion, qui permet de réaliser une impaction de l'insert dans la cupule avec une grande précision de l'orientation relative de l'insert et de la cupule, évitant ainsi dans une très grande mesure une détérioration sur le long terme, notamment en évitant fêlures ou cassures, et garantissant ainsi une longue durée de vie de la cupule dans la hanche.

Suivant l'invention, un dispositif d'insertion d'un insert, notamment en céramique, par impaction dans une cupule destinée à être reçue dans le cotyle d'une hanche, comportant:
- des moyens de préhension de l'insert, notamment au niveau d'un bord libre supérieur de l'insert ;
- une tige d'application de choc destinée à la prise du dispositif par le chirurgien et à l'application d'un choc d'impaction à l'insert lorsqu'il est tenu par les moyens de préhension en face de la partie creuse de la cupule pour l'y introduire; et
- une tête d'impaction ayant une surface destinée à venir en contact avec au moins une partie de l'insert, notamment la surface intérieure d'un creux de l'insert,
est caractérisé en ce que
- la tête d'impaction et la tige d'application sont liées l'une à l'autre d'une manière solidaire en rotation, notamment la tête est issue de la tige ; et
- l'ensemble tête d'impaction et tige est monté pivotant par rapport aux moyens de préhension de l'insert, notamment à la manière d'une rotule.

On obtient ainsi un dispositif d'insertion par impaction qui permet de réaliser cette insertion d'une manière particulièrement fiable et simple, en diminuant dans une très grande mesure le risque d'une insertion « décentrée », source potentielle de rupture ou fêlure de la cupule une fois posée dans le cotyle. Quelque soit l'angle de la force réalisant l'impaction par rapport à l'insert, c'est à dire l'orientation de la tige par rapport à l'insert, ce dernier étant maintenu par le dispositif d'insertion avec possibilité de pivotement se positionne toujours parfaitement par rapport à la cupule, quelque soit l'orientation de la tige par rapport à la cupule, de sorte que le chirurgien n'a plus besoin, comme dans l'art antérieur, de s'assurer que la tige arrive parfaitement perpendiculaire au plan de base de la cupule lors de l'insertion.

De préférence, le point pivot du montage pivotant des moyens de préhension par rapport à l'ensemble tige-tête d'impaction se trouve sensiblement au centre de la sphère intérieure de l'insert.

Suivant un mode de réalisation préféré de l'invention, les moyens de préhension de l'insert sont constitués d'une griffe de maintien comportant au moins deux, de préférence trois, pattes destinées à enserrer entre elles l'insert de manière libérable élastiquement.

De préférence, la griffe de maintien comporte une plaque de base de la périphérie de laquelle font saillie les pattes.

En particulier, la plaque de base est percée d'un trou, notamment sensiblement central, dont la surface de la paroi intérieure épouse la forme d'une rotule formée sur la tige de manière à permettre le pivotement relatif de la tige et de la plaque de base.

Suivant un mode de réalisation avantageux, l'extrémité distale de la tige comporte une tête faisant saillie latéralement de la tige et constituant la tête d'impaction, la tête ayant une surface d'extrémité distale, notamment en forme de dôme, destinée à épouser en partie la forme du fond de la cavité intérieure de l'insert.

De préférence, la surface extérieure de la tête 9 d'impaction destinée à s'appliquer contre la paroi de fond de la cavité 4 est inscrite dans une sphère dont le centre coïncide avec le point pivot.

On obtient ainsi une orientation libre de l'insert.

Suivant un perfectionnement constituant également une invention en tant que tel, indépendamment de l'invention décrite ci dessus, le dispositif d'insertion d'un insert, notamment en céramique, dans une cupule destinée à être reçue dans le cotyle d'une hanche, comportant des moyens de préhension de l'insert, notamment au niveau d'un bord libre supérieur de l'insert, et une tige destinée à l'impaction de l'insert lorsqu'il est tenu par les moyens de préhension en face de la partie creuse de la cupule, comporte des moyens pour impartir un choc à la tige vers le bas pour l'impaction de l'insert dans la cupule, c'est à dire l'introduire à adaptation en force à l'intérieur de la cupule, ces moyens pour impartir un choc impartissant à la tige un choc ayant une puissance déterminée à l'avance.

En fonction des données et/ou de l'expérience, on peut ainsi adapter la puissance du choc pour optimiser le coup donné à l'insert pour l'insérer, évitant ainsi des variations de la puissance en fonction du praticien et/ou de sa forme du jour susceptible d'entraîner soit une fêlure ou cassure de l'insert ou de la cupule, soit une insertion incomplète de l'insert dans la cupule.

Suivant un mode de réalisation préféré de l'invention, les moyens pour impartir un choc de puissance déterminée à l'avance à la tige comportent un piston et un ressort.

De préférence il est prévu des moyens pour déplacer le piston à l'encontre de la compression du ressort de manière à comprimer le ressort.

Suivant un mode de réalisation préféré, on peut prévoir des moyens pour bloquer le piston dans une position chargée dans laquelle le ressort est comprimé et des moyens, notamment sous la forme d'une détente, destinés à libérer le piston, la libération du piston entraînant son déplacement instantané par la décompression du ressort et son action d'impulsion sur la tige impartissant à cette dernière un choc de puissance déterminée à l'avance.

De préférence, le ressort et le piston sont reçus dans une cavité proximale à la tige.

De préférence, la cavité recevant le piston et le ressort est formé dans un châssis d'une pièce avec la tige.

De préférence une poignée, notamment de type crosse à pistolet fait saillie latéralement du châssis.

La présente invention se rapporte également à un ensemble comportant un dispositif d'insertion suivant l'invention et au moins un insert, notamment en céramique, destiné à être inséré dans une cupule de cotyle, ainsi qu'à un assemblage comportant un ensemble suivant l'invention et une cupule destinée à être reçue dans un cotyle de hanche.

On décrit maintenant, à titre d'exemple, des modes de réalisation de l'invention en se reportant aux dessins, dans lesquels:
- la figure 1 représente un dispositif d'insertion suivant l'invention, une cupule et un insert en céramique destiné à être inséré par le dispositif d'insertion à l'intérieur de la cupule 50;
- les figures 2A, 2B et 2C représentent les différentes étapes de l'insertion de l'insert à l'aide du dispositif d'insertion dans la cupule ;
- la figure 3 est une vue en perspective de dessous d'une partie du dispositif d'insertion de la figure 1 ; et
- la figure 4 est une vue en perspective d'un dispositif d'application d'un choc à la tige du dispositif d'insertion des figures précédentes qui, suivant un mode de réalisation avantageux, peut être adapté au dispositif d'insertion des figures 1 à 3.

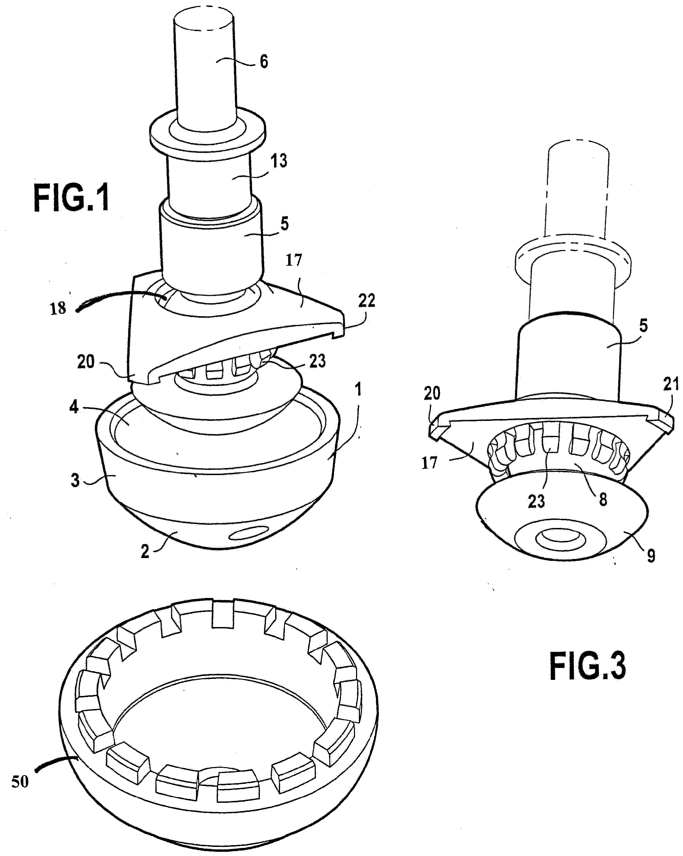

A la figure 1, il est représenté suivant une vue en perspective un dispositif d'insertion suivant un mode de réalisation de l'invention d'un insert 1 en matériau céramique dans une cupule 50. L'insert 1 en céramique est constitué d'un corps en céramique, notamment en céramique d'alumine ayant une surface extérieure en forme de calotte 2 sphérique terminée par une partie 3 de bord supérieur ou proximal de forme légèrement tronconique. L'insert est creux et comporte une cavité 4 intérieure dont la paroi est de forme hémisphérique. En particulier, le cône inscrit de la partie tronconique 3 de bord de la surface extérieure de l'insert fait un angle d'environ 3 à 5° par rapport à l'axe vertical de symétrie de l'insert en céramique.

Le dispositif d'insertion est constitué d'une tige 6 cylindrique circulaire à laquelle a été adapté à son extrémité distale un embout 5 d'impaction. L'embout 5 comporte un tronçon proximal tubulaire 7 taraudé pour recevoir en son sein l'extrémité filetée de la tige 6, pour ainsi les solidariser par vissage. On pourrait, à la place, prévoir que l'embout soit maintenu avec la tige à l'aide d'un autre type de liaison, par exemple un système d'encliquetage. On pourrait aussi prévoir que l'embout soit monobloc avec la tige.

L'embout 5 comporte ensuite un tronçon intermédiaire en forme de bille 8 sphérique, puis une tronçon distal formant tête 9 d'impaction. La tête 9 d'impaction est de plus grande dimension transversale que le reste de l'embout 5, notamment que le tronçon 7 proximal, et sa surface inférieure ou distale est en forme de dôme en étant au moins en partie complémentaire de la surface intérieure de la cavité 4 de l'insert 1, de manière à pouvoir réaliser avec celle ci en contact de surface. On pourrait prévoir cependant, à l'inverse que La tête 9 d'impaction soit de plus petite dimension transversale que le reste de l'embout 5, notamment que le tronçon 7 proximal.

La surface extérieure de la tête 9 destinée à s'appliquer contre la paroi de fond de la cavité 4 est inscrite dans une sphère, concentrique avec la sphère définissant la bille 8. Cela facilite la libre orientation de l'insert.

A l'intérieur de l'embout 5, il est formé au niveau de la fin du tronçon 7 proximal, une butée 10 contre laquelle vient buter l'extrémité distale de la tige 6, pour en bloquer son vissage avec le taraudage interne du tronçon 7. En outre, un canal 11 débouchant à la surface inférieure en forme de dôme du tronçon 9 par une ouverture facilitant le nettoyage interne de l'embout.

Un élément 13 de raccordement également tubulaire termine la liaison entre l'embout 5 et la tige 6. Cet élément 13 comporte une extrémité taraudée qui se visse dans la partie du filetage de la vis qui ne coopère pas avec le taraudage de l'embout 5. De l'autre côté, proximal, l'élément 13 est soudé en 14 à la tige.

Un élément formant griffe est monté pivotant par rapport à la bille 8. Cette griffe comporte une plaque 17 sensiblement plane, de forme triangulaire et aux trois sommets de laquelle font saillies trois pattes 20, 21 et 22 respectives. Les pattes sont destinées à enserrer l'insert au niveau de son bord supérieur ou proximal. Les pattes et/ou la plaque sont en un matériau et/ou ont une épaisseur tels qu'elles présentent une certaine élasticité leur permettant d'enserrer élastiquement le bord supérieur de l'insert 1 de manière élastique et de le relâcher lorsque qu'une force de déformation est appliquée à la griffe, par exemple en appuyant sur la plaque 17. La plaque 17 est percée en son milieu d'un trou 18 de forme complémentaire de la bille 8 sphérique formant rotule et comporte des éléments 23 en forme de dents sensiblement de forme complémentaire de celle de la bille 8 sphérique de manière à en épouser la forme. Le matériau et/ou l'épaisseur des dents est(sont) choisi(s) de manière à permettre l'encliquetage élastique de la plaque sur la rotule. le bord intérieur du trou 18 a une paroi intérieure de forme incurvée complémentaire de la forme de la bille 8 formant rotule. Le montage est tel que la plaque 17, une fois encliquetée par les dents 23 à la rotule 8, peut pivoter par rapport à la rotule 8, et donc par rapport à la tige 6 solidaire, notamment en rotation, de la rotule 8.

Dans le mode de réalisation représenté, on a prévu trois pattes. Cependant, on pourrait en prévoir plus, notamment quatre ou cinq pour des inserts de grande taille. On pourrait aussi à la place des pattes prévoir une galette faisant tout le tour.

La dimension de la plaque est réalisée de telle manière que lorsque la tête 9 vient en contact contre la surface de la paroi intérieure de l'insert en céramique, les trois pattes 20, 21 et 22 enserrent le bord périphérique libre de la partie 3 tronconique de l'insert 1 en céramique, les trois pattes 20, 21 et 22 s'étendant alors vers la cupule le long de la surface extérieure de la partie 3 de l'insert 1 en céramique en enserrant ce dernier. L'insert 1 en céramique est ainsi pris par la griffe de manière élastique.

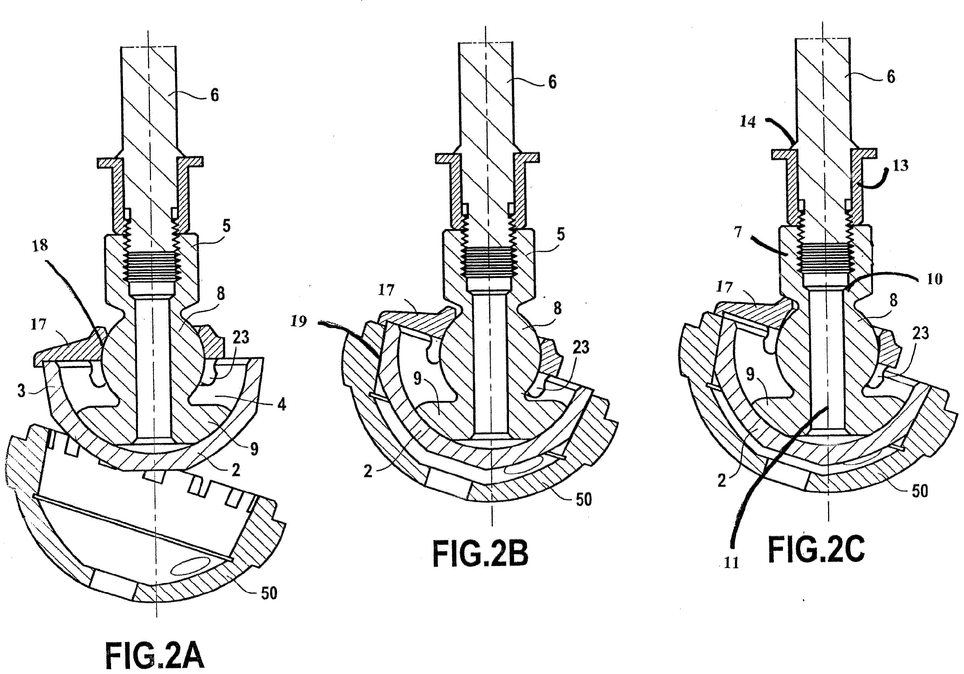

Une fois l'insert pris entre les pattes 20, 21, 22 (Figure 2A), on positionne à l'aide du dispositif l'insert 1 à l'intérieur de la cupule (Figure 2B). Cette étape se fait sans qu'il soit besoin que l'axe d'insertion (l'axe de la tige) soit perpendiculaire au plan de base ou supérieur de la cupule ou de l'insert, grâce au montage en pivotement relatif de l'ensemble griffe-insert préalablement solidarisé mutuellement par serrage des pattes. Cette deuxième étape de poussée a lieu jusqu'à ce que les bords distaux des pattes 20, 21 et 22 viennent buter contre le bord libre supérieur de la cupule. A ce moment, l'insert est en grande partie introduit dans la cupule en ayant sa partie 3 tronconique presque en contact avec la face interne de la cupule, dans une section 19 haute ayant une forme complémentaire de cet élément tronconique de l'insert. Les deux parties tronconiques de l'insert et de la cupule sont donc maintenues dans une position coaxiale mais sans solidarisation puisque les pattes maintiennent un jeu minimum entre l'insert et la cupule. Il en résulte que la simple poussée de l'insert dans la cupule en vient à être arrêtée à peu près au même moment que les extrémité distales des pattes 20, 21, 22 viennent en contact avec le bord libre de la cupule.

Ensuite, pour insérer de force l'insert dans la cupule, le chirurgien va appliquer un choc sur la tige, par exemple en donnant un coup sur le dessus de la tige vers le bas, dans la direction d'extension de la tige. Compte tenu de la souplesse des pattes et/ou de la plaque, ce choc va simultanément enfoncer de force l'insert dans sa position finale dans la cupule (position représentée à la figure 2C) et libérer les pattes de leur emprise sur l'insert.

Comme l'insert a été positionné grâce au dispositif de l'invention, parfaitement centré par rapport à la cupule dans la position de la figure 2B avant application du choc, il n'est pas nécessaire que la tige soit parfaitement perpendiculaire au plan supérieur de la cupule et de l'insert (plans dans lesquels s'étendent respectivement les bords libres de la cupule et de l'insert) lors de l'application de ce choc, l'angle entre la tige et l'axe de l'insert peut en particulier, comme représenté à la figure 2C, avoir une valeur élevée, par exemple 10 à 20°, sans que cela n'ait la moindre incidence sur la « qualité » de l'insertion finale à force de l'insert.

Le blocage relatif de l'insert dans la cupule est réalisé par la légère différence de conicité des parois intérieure 19 de la cupule et 3 extérieure de l'insert 1. Cependant, on pourrait prévoir d'autres manière de réaliser cette adaptation en force, par exemple en prévoyant des saillies de la surface extérieure de l'insert.

Ci dessus, on a décrit un premier mode de réalisation dans lequel le chirurgien applique le choc d'impaction en donnant un coup sur la tige.

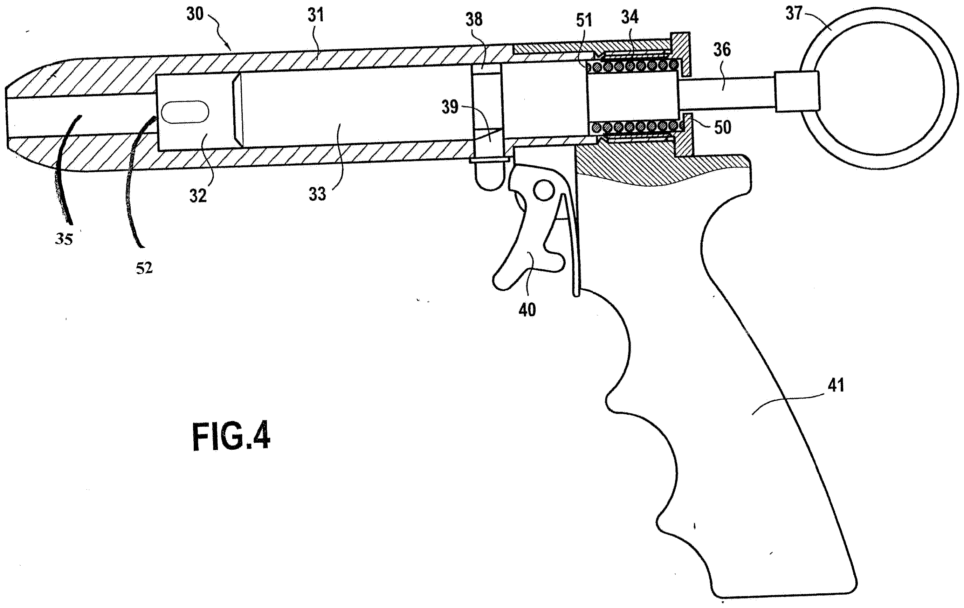

On peut, suivant un autre mode de réalisation avantageux, prévoir à l'extrémité proximale de la tige 6 une partie en forme de pistolet. Cette partie en forme de pistolet comporte un châssis 30 comportant un canon 31 destiné à être relié, notamment de manière solidaire, à la tige 6. Dans le canon 31, il est formé une cavité 32 dans laquelle est monté mobile en translation un piston 33 le long de l'axe du canon. Un ressort 34 est disposé à l'arrière du piston. Une tige 36 solidaire du piston 33 passe à travers le ressort et sort du châssis 30 par une ouverture 50 arrière. La tige 36 permet à l'utilisateur, notamment par l'intermédiaire d'un anneau 37 solidaire de la tige 36, de tirer le piston 33 vers l'arrière en comprimant ainsi le ressort 34 contre un épaulement 51 du piston 33 jusqu'à ce qu'une encoche circulaire 38 transversale vienne se bloquer par encliquetage dans une butée 39. Lorsque la butée 39 est bloquée dans l'encoche 38, le piston est déplacé le plus en arrière possible et le ressort 34 est comprimé. La butée 39 est montée rotative, de sorte qu'une queue de détente 40, montée rotative, entraîne la rotation de la butée 39 et donc sa sortie de l'encoche 38 et in fine la libération du piston 33. Sous l'effet de la charge du ressort pré-comprimé, le piston 33 se déplace alors à grande vitesse vers la gauche à la figure 4 et vient frapper l'extrémité distale de la cavité 32, entrant en collision avec la tige 6, dont l'extrémité proximale a été préalablement introduite dans un canal 35 d'introduction jusqu'au niveau de l'ouverture 52 distale de la cavité 32. Des moyens d'arrêt de l'introduction de la tige dans le canon 31 peuvent être prévus pour que la tige pénètre dans le canal 35 mais s'arrête à l'entrée 52 de la cavité 32. Ces moyens d'arrêt peuvent par exemple être une butée circulaire faisant saillie latéralement de la tige et venant buter contre l'ouverture distale de sortie du canal 35.

La tige subit alors un choc qui impacte l'insert en céramique pour l'insérer dans la cupule.

Le dispositif comporte également une poignée 41 en forme de crosse de pistolet.

Ainsi, la force d'impaction appliquée à la tige 6 peut être déterminée à l'avance par le choix du ressort 34, de sa raideur et de la course de déplacement vers l'arrière du piston 33. On s'assure ainsi d'une force d'impulsion constante sur la tige et par conséquent d'une force ou puissance d'impaction de l'insert dans la cupule qui est constante ou sensiblement constante, correspondant exactement à ce qui est nécessité pour assurer une bonne insertion de l'insert, sans pour autant que ce dernier ne soit fêlé, comme c'était le cas dans l'art antérieur où il était difficile de doser l'effort du marteau. La vitesse impartie à la tige par la libération du piston peut être déterminée à l'avance par le choix de la géométrie des éléments constitutifs du dispositif d'insertion, notamment du ressort, du piston, de la longueur du tube 6 et analogue.

## Revendications

1. Dispositif d'insertion d'un insert (1), notamment en céramique, par impaction dans une cupule (50) destinée à être reçue dans le cotyle d'une hanche, comportant :
- des moyens (17, 20, 21, 22) de préhension de l'insert;
- une tige (5, 6) d'application de choc destinée à la prise du dispositif par le chirurgien et à l'application d'un choc d'impaction à l'insert lorsqu'il est tenu par les moyens de préhension en face de la cupule pour l'y introduire ; et
- une tête (9) d'impaction ayant une surface destinée à venir en contact avec au moins une partie de l'insert,
**caractérisé en ce que**
- la tête (9) d'impaction et la tige (5, 6) d'application sont liées l'une à l'autre d'une manière solidaire en rotation ; et
- l'ensemble tête d'impaction et tige est monté pivotant par rapport aux moyens de préhension de l'insert.

2. Dispositif suivant la revendication 1, **caractérisé en ce que** le point pivot du montage pivotant des moyens de préhension par rapport à l'ensemble tige-tête d'impaction se trouve sensiblement au centre de la sphère définissant la partie hémisphérique intérieure de l'insert.

3. Dispositif suivant la revendication 1 ou 2, **caractérisé en ce que** la surface extérieure de la tête (9) d'impaction destinée à s'appliquer contre la paroi de fond de la cavité (4) est inscrite dans une sphère dont le centre coïncide avec le point pivot.

4. Dispositif suivant la revendication 1, 2 ou 3, **caractérisé en ce que** les moyens de préhension de l'insert sont constitués d'une griffe de maintien comportant au moins deux, notamment de trois à cinq, pattes (20, 21, 22) destinées à enserrer entre elles l'insert de manière libérable élastiquement.

5. Dispositif suivant la revendication 4, **caractérisé en ce que** la griffe de maintien comporte une plaque (17) de base de la périphérie de laquelle font saillie les pattes.

6. Dispositif suivant l'une des revendications 1 à 5, **caractérisé en ce que** le matériau et/ou l'épaisseur des pattes et/ou de la plaque est tel(le) que le choc va simultanément enfoncer de force l'insert dans sa position finale dans la cupule et libérer les pattes de leur emprise sur l'insert.

7. Dispositif suivant la revendication 5 ou 6, **caractérisé en ce que** la plaque (17) de base est percée d'un trou (18), notamment sensiblement central, dont la surface de la paroi intérieure épouse la forme d'une rotule (8) formée sur la tige de manière à permettre le pivotement relatif de la tige et de la plaque de base.

8. Dispositif suivant l'une des revendications 1 à 7, **caractérisé en ce que** l'extrémité distale de la tige comporte une tête faisant saillie latéralement de la tige et constituant la tête d'impaction, la tête ayant une surface d'extrémité distale, notamment en forme de dôme, destinée à épouser en partie la forme du fond de la cavité intérieure de l'insert.

9. Dispositif suivant l'une des revendications 1 à 8, **caractérisé en ce que** il est prévu des moyens pour impartir à la tige un choc vers le bas pour impacter l'insert dans la cupule, c'est à dire l'introduire à adaptation en force à l'intérieur de la cupule, ces moyens pour impartir impartissant à la tige un choc dont la puissance a une valeur déterminée à l'avance.

10. Dispositif suivant l'une des revendications précédentes, **caractérisé en ce que** les moyens (17, 20, 21, 22) de préhension sont des moyens de préhension au niveau d'un bord libre supérieur de l'insert.

11. Dispositif suivant l'une des revendications précédentes, **caractérisé en ce que** la surface destinée à venir en contact de la tête (9) vient en contact avec la surface intérieure d'un creux de l'insert.

12. Dispositif suivant l'une des revendications précédentes, **caractérisé en ce que** la tête (9) est issue de la tige (5,6).

13. Dispositif suivant l'une des revendications précédentes, **caractérisé en ce que** l'ensemble tête d'impaction et tige est monté pivotant par rapport aux moyens de préhension à la manière d'une rotule.

14. Ensemble comportant un dispositif d'insertion suivant l'une des revendications 1 à 13 et au moins un insert, notamment en céramique, destiné à être inséré dans une cupule de cotyle.

15. Assemblage comportant un ensemble suivant la revendication 14 et une cupule destinée à être reçue dans un cotyle de hanche.

## Patentansprüche

1. Vorrichtung zum Einsetzen eines Einsatzes (1), insbesondere aus Keramik, durch Impaktieren in eine Schale (50), die zur Aufnahme in der Gelenkpfanne einer Hüfte bestimmt ist, umfassend:
- Mittel (17, 20, 21, 22) zum Greifen des Einsatzes;
- einen Stab (5, 6) zur Schlagabgabe, der zum Greifen der Vorrichtung durch den Chirurgen und zum Abgeben eines Impaktierschlags auf den Einsatz bestimmt ist, wenn er durch die Greifmittel gegenüber der Schale gehalten wird, um ihn dort einzuführen; und
- einen Impaktierkopf (9), der eine Fläche aufweist, die zum Inkontaktkommen mit mindestens einem Teil des Einsatzes bestimmt ist,
**dadurch gekennzeichnet, dass**
- der Impaktierkopf (9) und der Schlagabgabestab (5, 6) drehfest miteinander verbunden sind; und
- die Impaktierkopf- und Stabanordnung in Bezug auf die Greifmittel des Einsatzes drehbar gelagert ist.

2. Vorrichtung nach Anspruch 1, **dadurch gekennzeichnet, dass** sich der Drehpunkt der drehbaren Lagerung der Greifmittel in Bezug auf die Stab-/Impaktierkopfanordnung im Wesentlichen in der Mitte der Kugel befindet, die den halbkugelförmigen inneren Teil des Einsatzes definiert.

3. Vorrichtung nach Anspruch 1 oder 2, **dadurch gekennzeichnet, dass** die Außenfläche des Impaktierkopfs (9), die zur Anlage an die Bodenwand des Hohlraums (4) bestimmt ist, die Form einer Kugel aufweist, deren Mitte mit dem Drehpunkt übereinstimmt.

4. Vorrichtung nach Anspruch 1, 2 oder 3, **dadurch gekennzeichnet, dass** die Greifmittel des Einsatzes aus einer Haltebacke bestehen, die mindestens zwei, insbesondere drei bis fünf Krallen (20, 21, 22) umfasst, die dazu bestimmt sind, den Einsatz auf elastisch lösbare Weise zwischen sich einzuschließen.

5. Vorrichtung nach Anspruch 4, **dadurch gekennzeichnet, dass** die Haltebacke eine Grundplatte (17) umfasst, von deren Rand die Krallen vorspringen.

6. Vorrichtung nach einem der Ansprüche 1 bis 5, **dadurch gekennzeichnet, dass** das Material und/oder die Dicke der Krallen und/oder der Platte derart ist/sind, dass der Schlag den Einsatz in seine Endposition in der Schale hineinpresst und gleichzeitig die Krallen von ihrem Eingriff in den Einsatz lösen wird.

7. Vorrichtung nach Anspruch 5 oder 6, **dadurch gekennzeichnet, dass** die Grundplatte (17) eine Bohrung (18) aufweist, die insbesondere im Wesentlichen mittig ist, wobei die Fläche der Innenwand an die Form eines Kugelgelenks (8) angepasst ist, das auf dem Stab ausgebildet ist, um die relative Drehung des Stabs und der Grundplatte zu ermöglichen.

8. Vorrichtung nach einem der Ansprüche 1 bis 7, **dadurch gekennzeichnet, dass** das distale Ende des Stabs einen Kopf umfasst, der seitlich von dem Stab vorspringt und den Impaktierkopf bildet, wobei der Kopf eine distale Endoberfläche, insbesondere in Kuppelform aufweist, die dazu bestimmt ist, sich teilweise der Form des Bodens des inneren Hohlraums des Einsatzes anzupassen.

9. Vorrichtung nach einem der Ansprüche 1 bis 8, **dadurch gekennzeichnet, dass** Mittel zum Übertragen eines nach unten gerichteten Schlags auf den Stab vorgesehen sind, um den Einsatz in die Schale zu impaktieren, das heißt, ihn mittels Presspassung ins Innere der Schale einzusetzen, wobei diese Übertragungsmittel auf den Stab einen Schlag übertragen, dessen Kraft einen vorbestimmten Wert aufweist.

10. Vorrichtung nach einem der vorhergehenden Ansprüche, **dadurch gekennzeichnet, dass** es sich bei den Greifmitteln (17, 20, 21, 22) um Greifmittel in Höhe eines freien Rands oberhalb des Einsatzes handelt.

11. Vorrichtung nach einem der vorhergehenden Ansprüche, **dadurch gekennzeichnet, dass** die Fläche, die dazu bestimmt ist, mit dem Kopf (9) in Kontakt zu kommen, mit der Innenfläche einer Vertiefung des Einsatzes in Kontakt kommt.

12. Vorrichtung nach einem der vorhergehenden Ansprüche, **dadurch gekennzeichnet, dass** der Kopf (9) aus dem Stab (5, 6) hervorgeht.

13. Vorrichtung nach einem der vorhergehenden Ansprüche, **dadurch gekennzeichnet, dass** die Impaktierkopf- und Stabanordnung in Bezug auf die Greifmittel nach Art eines Kugelgelenks drehend montiert ist.

14. Anordnung, die eine Vorrichtung zum Einsetzen nach einem der Ansprüche 1 bis 13 und mindestens einen, insbesondere keramischen, Einsatz umfasst, der zum Einsetzen in die Schale einer Gelenkpfanne bestimmt ist.

15. Baugruppe, die eine Anordnung nach Anspruch 14 und eine Schale umfasst, die zur Aufnahme in einer Hüftgelenkpfanne bestimmt ist.

## Claims

1. Device for inserting an insert (1), made in particular of ceramic material, by impaction into a cup (50) designed to be received in the cotyl of a hip, comprising:
- means (17, 20, 21, 22) for gripping the insert, in particular at an upper free edge of the insert;
- a rod (5, 6) for imparting shock designed to be held by the surgeon and for the application of an impaciton shock to the insert when it is held by gripping means opposite the cup to be introduced therein; and
- an impaction head (9) with a surface designed to come into contact with at least one portion of the insert, in particular the inner surface of a hollow of the insert,
**characterised in that**
- the impaction head (9) and the application rod (5, 6) are connected to one another so as to be joined in rotation, in particular the head emerges from the rod; and
- the head and rod assembly is mounted pivotably in relation to the insert gripping means.

2. Device according to claim 1, **characterised in that** the pivot point of the assembly pivoting the gripping means relative to the rod-impaction head assembly is located substantially in the centre of the sphere defining the inner hemispherical part of the insert.

3. Device according to claim 1 or 2, **characterised in that** the external surface of the impaction head (9) designed to be applied against the base wall of the cavity (4) is in the form of a sphere, the centre of which coincides with the pivot point.

4. Device according to claim 1, 2 or 3, **characterised in that** the gripping means of the insert are formed by a holding grip comprising at least two, in particular three to five, feet (20, 21, 22) designed to fit around the insert in a elastically releasable manner.

5. Device according to claim 4, **characterised in that** the holding grip comprises a base plate (17) from the periphery of which the feet project.

6. Device according to one of claims 1 to 5, **characterised in that** the material and/or the thickness of the feet and/or the plate is such that the shock simultaneously drives the insert into its final position in the cup by force and releases the feet from their grip on the insert.

7. Device according to claim 5 or 6, **characterised in that** the base plate (17) is pierced by a hole (18), in particular substantially in the centre, the inner wall surface of which matches the form of a pivot pin (8) formed on the rod in such a way as to enable the relative pivoting of the rod and the base plate.

8. Device according to any one of claims 1 to 7, **characterised in that** the distal end of the rod comprises a head projecting laterally from the rod and forming the impaction head, the head having a distal end surface, in particular in the form of a dome, designed to match in part the form of the base of the inner cavity of the insert.

9. Device according to one of claims 1 to 8, **characterised in that** means are provided for imparting to the rod a downwards shock to impact the insert into the cup, that is to introduce it with an adjustment of force to the interior of the cup, said means imparting a shock to the rod of predetermined strength.

10. Device according to one of the preceding claims, **characterised in that** said gripping means (17, 20, 21, 22) are means for gripping at the level of an upper free edge of said insert.

11. Device according to one of the preceding claims, **characterised in that** said surface designed to come into contact of said head (9) comes into contact with the internal surface of a cavity of said insert.

12. Device according to one of the preceding claims, **characterised in that** said head (9) is coming from said rod (5, 6).

13. Device according to one of the preceding claims, **characterised in that** the assembly made of said impaction head and of said rod is mounted pivotably in relation to said insert gripping means in the manner of a pivot pin.

14. Assembly comprising an insertion device according to one of claims 1 to 13 and at least one insert, made in particular of ceramic material, designed to be inserted into a cotyl cup.

15. Unit comprising an assembly according to claim 14 and a cup designed to be received in a cotyl of a hip.
